# EUROPEAN PATENT SPECIFICATION

(11) **EP 2 846 817 B1**
(45) Date of publication and mention of the grant of the patent: **17.10.2018**
(21) Application number: 12722077.0
(22) Date of filing: 11.05.2012
(51) Int. Cl.: A61K 38/16, A61K 39/08

(54) **TRANSPLANTATION OF MODIFIED MONOCYTES**
TRANSPLANTATION VON MODIFIZIERTEN MONOZYTEN
TRANSPLANTATION DES MONOCYTES MODIFIÉS

(43) Date of publication of application: 18.03.2015
(73) Proprietor: Otto-von-Guericke-Universität Magdeburg, 39106 Magdeburg (DE)
(72) Inventor: HEROLD, Joerg, 39104 Magdeburg (DE); BRAUN-DULLAEUS, Rüdiger C., 39114 Magdeburg (DE)
(74) Representative: Schrell, Andreas
(86) International application number: PCT/EP2012/002024
(87) International publication number: WO 2013/167156

(56) References cited:
- WO-A1-2008/055354
- WO-A2-2004/050855
- US-A1- 2004 191 215
- JOERG HEROLD ET AL: "Isolation and transduction of monocytes: promising vehicles for therapeutic arteriogenesis", LANGENBECK'S ARCHIVES OF SURGERY, SPRINGER, BERLIN, DE, vol. 391, no. 2, 1 April 2006 (2006-04-01) , pages 72-82, XP019343961, ISSN: 1435-2451, DOI: 10.1007/S00423-006-0033-9
- JOERG HEROLD ET AL: "Transplantation of Monocytes: A Novel Strategy for In Vivo Augmentation of Collateral Vessel Growth", HUMAN GENE THERAPY, vol. 15, no. 1, 1 January 2004 (2004-01-01), pages 1-12, XP055054825, ISSN: 1043-0342, DOI: 10.1089/10430340460732517

## Description

The present invention relates to antigen-loaded antigen-presenting cells (APC) for use in a therapeutic method for augmenting collateral vessel growth in a subject in need thereof and pharmaceutical compositions comprising antigen-loaded antigen-presenting cells.

WO 2004/050855 and WO 2008/055354 disclose a pharmaceutical composition comprising antigen-loaded antigen-presenting cells. US 2004/191215 Herold et al., Langenbeck's Arch Surg 2006 (391), 72-82, and Herold et al., Human Gene Therapy 2004 (15), 1-12, disclose the attraction of antigen-presenting cells for use in promoting arterogenesis.

In the case of vascular disease, the development of collateral circulation is directly associated with increased tissue viability and reduced long-term mortality. Thus, therapeutic approaches to augment collateralisation are of great interest, especially considering that 20% of patients with vascular disease are not suitable for current treatments such as percutaneous coronary intervention (PCI) or coronary artery bypass graft (CABG). Cell transplantation to augment local vessel growth or tissue repair has been explored to treat ischemic diseases. Such studies largely exploited the therapeutic potential of embryonic stem cells or progenitor cells, which inter alia may have a tumorigenic potential and further may even give rise to bioethical objections.

Collateral vessel growth (hereinafter also termed arteriogenesis) is driven by local inflammation. In diseased or injured tissue, inflammation occurs as the pressure gradient created by a stenosed or occluded conductance artery increases mechanical stress in preformed arterioles that bypass the diseased vessel. This inflammation response includes endothelial activation and the recruitment of leukocytes, mainly monocytes (hereinafter also termed MOs); these monocytes, in turn, create a highly arteriogenic environment by secreting multiple growth factors that induce remodelling of the arteriole into a functional collateral artery. Previously, it was demonstrated that transplantation of allogeneic, that means genetically different, MOs strongly promotes arteriogenesis in rabbits, most likely by enhancing local inflammation and amplifying recipient MO recruitment, whereas transplantation of autologous, that means genetically identical, MOs does not mild. Although this finding suggested a novel therapeutic direction to promote vessel growth, conducting allogeneic cell transplantation without immune suppression poses serious risks including transmission of hepatitis and HIV.

Thus, the technical problem underlying the present invention is the provision of means and methods allowing an improved prophylaxis and therapy of vascular diseases, in particular providing means and methods to cure vascular diseases, in particular occlusive diseases in a manner overcoming the above-identified technical problems and disadvantages.

The present invention solves its underlying technical problem by the provision of an antigen-loaded antigen-presenting cell (the term APC refers to antigen-presenting cell) for use in a therapeutic method for augmenting collateral vessel growth, namely for treating a disease selected from the group consisting of coronary artery diseases, cerebral-occlusive diseases, peripheral-occlusive diseases, visceral-occlusive diseases, renal artery diseases and mesenterial arterial insufficiency in a subject in need thereof, wherein the subject in need thereof is a subject being immunised against said antigen.

The present invention provides the teaching to provoke a local inflammation by systemic application of TT engineered cells in a subject in need thereof according to which APCs, preferably monocytes, in particular autologous or syngeneic monocytes, are engineered ex vivo, hereinafter also termed in vitro, to present an antigen, preferably tetanus toxoid (TT) antigen. Thus, the APCs become antigen-loaded, e.g. by exposing the APCs prior to transplantation to said antigen. The obtained antigen-loaded APCs are for use to be transplanted into a subject in need thereof, which subject was prior to transplantation immunised against said antigen. The antigen-loaded APSs elicit a strong antigen response and induce collateral growth in antigen-immunised, preferably TT-immunized, subjects. This teaching provides a novel therapeutic approach to augment collateral vessel growth using APCs, preferably monocytes, preferably syngeneic or autologous monocytes.

Accordingly, the present invention provides a cellular-based therapy to augment arteriogenesis in the course of which APCs, preferably autologous or syngeneic MOs, are engineered to induce a local immunologic reaction that in turn augments collateral growth. The present invention shows that the immunoregulatory capacity of transplanted APC engineered autologous and syngeneic MOs is associated with an enormous restoration damages of blood vessels. The teaching is provided that to induce a transient inflammatory reaction by implanting stimulated APCs (e.g. TTsynMOs) it is advantageous to use a recipient that had been immunized for the antigen, e.g.TT. The present teaching demonstrates that with specific receptor modification, syngenic MOs can initiate the adaptive and initiate immune response and thereby augment collateral vessel growth.

Thus, it is shown that transplantation of ex vivo antigen-, in particular tetanus toxin-engineered syngenic MOs is able to augment arteriogenesis by triggering a natural immune response. Thus, the lymphocyte system contributes to collateral formation.

The present invention teaches that by triggering the immune system in a precise way to promote an inflammatory response, collateral arteries can be remodelled to a degree normally achieved only by using high amounts of cytokines such as MCP1, GM-CSF or LPS via a mini osmotic pump, which has systemic side effects.

The use of TT antigen provides in particular the following advantages: (1) TT vaccination is recommended and proven by the Centers for Disease Control and Prevention; (2) the interaction between TT vaccine and the immune system is one of the best characterized; (3) 90 % of all people have been immunized against TT, thus conferring them life-long protective immunity and enabling TT-engineered cells to be transplanted quickly, without the need to booster the immune system ahead of time; and (4) this life-long protective immunity is also ideal for repetitive cell transplantation to improve the restoration of blood flow during restenosis and progression in cardiovascular patients over years. The present teaching is e superior to current strategies implanting autologous MOs in CAD-patients or the intra muscular injection of growth factors. Also transdifferentiation of stem and progenitor cells could be bypassed by transplantation of differentiated MOs.

In the context of the present invention, the term "therapeutic method" means methods to prevent and cure states of disorder, malfunction, damage or disease of a subject in need thereof and therefore includes prophylactic and curative methods practised on the human or animal body. Preferably, the term "therapeutic" solely refers to curative methods.

In the context of the present invention, the term "antigen-loaded antigen-presenting cell" refers to an APC which presents at least one antigen on its cell surface and wherein said antigen is characterised as an antigen by its capability to elicit an immune response in a subject exposed to said antigen.

In the context of the present invention, the term "augmenting collateral vessel growth" is also meant to refer to "inducing or promoting or stimulating or activating" collateral vessel growth. The term "augmenting collateral vessel growth" is also meant to refer to augmenting "arteriogenesis". An augmented collateral vessel growth can be determined histologically and/or by measuring the blood flow, for instance by laser Doppler perfusion analysis.

In the context of the present invention, the term "a subject being immunised against said antigen" refers to a subject which has been exposed, in particular contacted, with the antigen such that the subject developed an immune response to said antigen and acquired a corresponding immune memory towards said antigen. The development of said immune response is measured by determining the titer of the antibody against said antigen found in the subject. Thus, the present antigen-loaded APC are to be administered to subjects which already developed an immune response and acquired an immune memory towards said specific antigen prior to the presently provided administration of an antigen-loaded antigen presenting cell.

The immunisation of the subject may be a general immunisation or an immunisation just prior to transplantation or both.

In the context of the present invention, the term "autologous" cell means a cell being collected from the same individual, that means subject, patient or recipient, on whom it will used, in particular transplanted. Thus, the donor and the recipient of an autologous cell is the same individual.

In the context of the present invention, the term "syngeneic" or "syngenic" cell means a cell a) being collected from an genetically identical, but different individual in comparison to the individual, that means subject, patient or recipient, receiving the cell and b) being genetically identical, or sufficiently identical and immunologically compatible as to allow for use, in particular transplantation, without eliciting an immune response in said recipient. Thus, the donor and the recipient of a syngeneic cell are not the same individuals but have the same genetic identity For example, a syngeneic cell is for transplantation from an identical twin or an individual from inbred mammal race.

In contrast, a cell being from another genetically different donor of the same species, is called "allogeneic". A "xenogeneic" transfer is transfer between different species.

In the context of the present invention, an antigen-presenting cell (APC) is a cell able to display foreign antigen complexes with major histocompatibility complex (MHC) on their surfaces. T-cells may recognize these complexes using their T-cell receptors (TCRs). Thus, these cells process antigens and present them to T-cells. T cells cannot recognize, and therefore react to, 'free' antigen. T cells can only recognize an antigen that has been processed and presented by cells via an MHC molecule.

APCs act by internalizing an antigen, either by phagocytosis or by receptor-mediated endocytosis, and then displaying a fragment of the antigen, bound to a class II MHC molecule, on their membrane. The T cell recognizes and interacts with the antigen-class II MHC molecule complex on the membrane of the antigen-presenting cell. An additional co-stimulatory signal is then produced by the antigen-loaded antigen-presenting cell, leading to activation of the T cell.

In a preferred embodiment, the present invention relates to the antigen-loaded APC according to the present invention, wherein the subject is a mammalian subject, for instance a horse, pig, camel, sheep, ape, monkey, dog, rat, mouse, cat or human, in particular human subject.

In a preferred embodiment of the present invention, the APC and the APCs are purified, preferably highly purified, in particular isolated APCs. In a preferred embodiment, the APCs have a purity of at least 85, preferably at least 90, preferably at least 91, preferably at least 92, preferably at least 93, preferably at least 94, preferably at least 95, preferably at least 96, preferably at least 97, preferably at least 98 or preferably at least 99%, based on the overall cell population.

In a preferred embodiment of the present invention, the APCs have a viability of at least 85, preferably at least 90, preferably at least 91, preferably at least 92, preferably at least 93, preferably at least 94, preferably at least 95, preferably at least 96, preferably at least 97, preferably at least 98 or preferably at least 99%.
In a preferred embodiment of the present invention, the APC is a differentiated cell.
In a preferred embodiment, the present invention relates to the antigen-loaded APC according to the present invention, wherein the APC is a monocyte (hereinafter termed also MO), a monocyte precursor, a B-cell or a dendritic cell.

In a preferred embodiment, the present invention relates to the antigen-loaded APC according to the present invention, wherein the antigen is preferably selected from a group consisting of antigens related to infectious diseases, preferably antigens to infectious diseases which are commonly used for vaccination and which are recommended by the Centers for Disease Control and Prevention. Preferably, the antigen is selected from the group consisting of tetanus toxoid (hereinafter also termed TT), diphtheria toxin, and pertussis toxin. Most preferred is the antigen tetanus toxoid.
In a preferred embodiment, the present invention relates to the antigen-loaded APC according to the present invention, wherein the antigen is not a cytokine, is not a growth factor or is not a hormone.

In a preferred embodiment, the present invention relates to the antigen-loaded APC according to the present invention, wherein the antigen is tetanus toxoid.

In a preferred embodiment of the present invention, an APC is loaded with an antigen to become an antigen-loaded APC by exposing the APC ex vivo to the antigen so as to allow internalisation, processing and surface-presentation of said antigen.

In a preferred embodiment of the present invention, an APC is loaded with an antigen to become an antigen-loaded APC by genetic engineering said cell, in particular by transfecting or transducing the APC with a virus or vector comprising a nucleotide sequence expressing the antigen, allowing expression and surface presentation of said antigen.

In a preferred embodiment, the present invention relates to the antigen-loaded APC according to the present invention, wherein said APCs are autologous or syngeneic to the subject.

Thus, the present invention in a preferred embodiment advantageously avoids the risk of transmission of e. g. hepatitis or AIDS by using, preferably highly purified, autologous or syngeneic APCs, preferably MO, that means cells having the same genetic background as the cells of the recipient.

In a preferred embodiment of the present invention, the APC provided for being loaded with the antigen is obtained from a subject, preferably donor, which is not and was not immunised against said antigen, thus, has never been in contact with said antigen.

In a particular preferred embodiment of the present invention, the APC provided for being loaded with the antigen is obtained from a subject, preferably donor, which is immunised against said antigen, thus, has been in contact with said antigen and developed an immune response against said antigen.

In a preferred embodiment, the present invention relates to the antigen-loaded APC according to the present invention, wherein the collateral vessel are collateral arteries or arteries from pre-existing arteriolar connections.

The present invention relates to the antigen-loaded APC according to the present invention, wherein the subject is suffering from an occlusive disease, preferably selected from the group consisting of a coronary heart disease (hereinafter also termed coronary artery disease) or a peripheral arteriosclerosis (hereinafter also termed peripheral-occlusive disease or peripheral vascular disease).

In a preferred embodiment the present invention relates to the antigen-loaded APC according to the present invention, wherein the subject is suffering from a coronary heart disease or a peripheral arteriosclerosis.

The present invention relates to the antigen-loaded APC according to the present invention, wherein the occlusive disease is selected from the group consisting of coronary artery diseases, cerebral-occlusive diseases, peripheral-occlusive diseases, visceral-occlusive diseases, renal artery diseases and mesenterial arterial insufficiency.

In a preferred embodiment, the present invention relates to the antigen-loaded APC according to the present invention, wherein the subject in need thereof is subjected or was subjected to an agent or radiation or surgical treatment damaging or destroying arteries.

The present invention also provides pharmaceutical composition comprising the antigen-loaded APC according to the present invention and a pharmaceutically acceptable carrier.

The present invention also provides a pharmaceutical composition for use in a therapeutic method of augmenting collateral vessel growth, namely for treating a disease selected from the group consisting of coronary artery diseases, cerebral-occlusive diseases, peripheral-occlusive diseases, visceral-occlusive diseases, renal artery diseases and mesenterial arterial insufficiencyin a subject in need thereof comprising an antigen-loaded APC, wherein the subject in need thereof is a subject being immunised against said antigen, and a pharmaceutical acceptable carrier.

The present invention also relates to a method for augmenting collateral vessel growth in a subject in need thereof, wherein an antigen-loaded antigen-presenting cell is administered to the subject in need thereof in an amount sufficient to augment collateral vessel growth in said subject and wherein the subject in need thereof is a subject being immunised against said antigen.

The present invention also relates to a method for augmenting collateral vessel growth in a subject in need thereof, comprising the following steps:
a) providing APCs from a donor, preferably which APCs can also be received from a host,
b) exposing ex vivo the APCs obtained in step a) to an antigen so as to obtain antigen-loaded APCs,
c) providing a recipient, that means the subject in need thereof, being immunised against said antigen and
d) administering the antigen-loaded antigen-presenting cells obtained in step b) to the immunised recipient provided in step c) in an amount sufficient to augment collateral vessel growth in said subject.

The invention foresees also that steps b) and c) identified above can be performed simultaneously or step b) subsequent to step c) or step c) subsequent to step b).

In a preferred embodiment of the present invention, prior to administering the antigen-loaded APCs it is foreseen to determine the immunisation stage of the recipient used in step c) and d), in particular to determine the antibody level to the antigen in said recipient. In case the immunisation stage is not sufficient, the recipient has to be immunised against said antigen. However, a subsequent immunisation of the subject is solely necessary, if the antibody level is too low.

Preferably, the donor is the same individual,that means the same patient, as the recipient. Thus, preferably the APC is autologous.

In another embodiment, the donor has the same genetic identity as the recipient. Thus, preferably the APC is syngeneic.

In a preferred embodiment of the present invention, the antigen-loaded APCs are administered to the subject in need thereof by transplantation. Thus, preferably the present APCs are for transplantation into a subject in need thereof.

Further preferred embodiments of the present invention are the subject matter of the subclaims.

The invention is described in more detail by way of the following nonlimiting examples and the accompanying figures.

The Figures show:

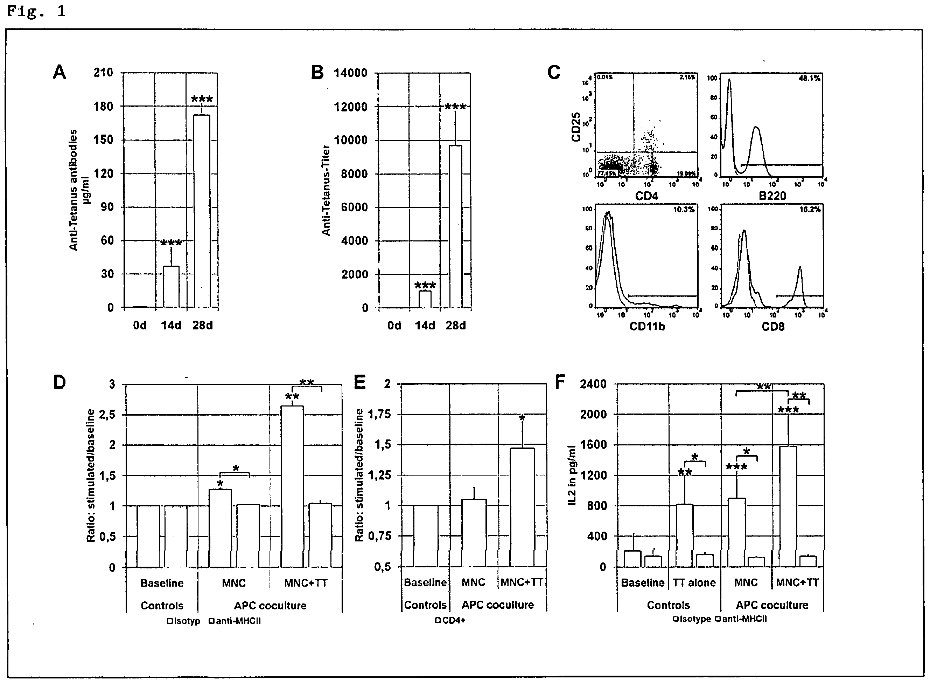

Fig. 1A ELISA determination of antibody titer showing massive increase 14 and 28 days after active immunization with tetanus toxoid.
Fig. 1B The level of serum monoclonal mouse-anti-tetanus-antibody increased in concentration compared with the wild-type control without pre-immunization, respectively levels of the mouse before receiving the immunisation. Results are the mean±s.d. of triplicates. Results are representative three experiments. *P < 0.05.
Fig. 1C FACS analysis from spleen derived leukocytes of immunized and non-immunized BALB/c mice revealed that the resulting cell suspension contained CD4, CD8, B-cells, MOs, polymorphonuclear leukocytes (PMNs) and CD4+ CD25+ cells.
Fig. 1D MTT T cell proliferation assay shows that TT-antigen presentation by TTsynMOs engineered MOs is MHC II related. Co-culture of TTsynMOs (100 µg/ml/24h) with spleen-derived T cells from immunized mice induced a 2.5 fold increase in proliferation of all T cells. Endogenous proliferation of non stimulated cells was the baseline for this experiment.

Co-cultivation of spleen-derived T cells from immunized mice with non-TT-engineered MNC induced mild T cell proliferation (25%) compared with the baseline. Results are the mean±s.d. of triplicates.

Results represent one of three similar experiments. Error bars represent means±s.d.. The endogenous effect of the MOs is explained by stimulation due to the isolation procedure and pre-stimulation with INF-y in the culture medium. This effect was also abolished by blocking the MHC II pathway using specific antibodies. Proliferation of T cells from immunized mice was not influenced by supplementation of tetanol pur into the medium.

Fig. 1E CFSE-assay quantification demonstrates co-cultivation of TTsynMOs induced a 1.5 fold increase in CD4 T-cells in comparison to baseline and non-engineered MOs in vitro.

Fig. 1F IL 2 production of spleen-derived T cells from immunized mice after stimulation with TTsynMOs or synMOs, or co-cultivation with tetanol pur. A 2 fold increase in IL2 levels was found after stimulation with TTsynMOs. Proliferation and cytokine secretion was abolished by incubation of co-cultures with functional anti-MHC class II receptor antibodies.

Fig. 2A Restoration of mouse hind limb perfusion assayed by laser Doppler measurement. Time points include before and directly after ligation, then one, two and three weeks post-ligation. The superiority of TTsynMO transplantation is shown graphically (Fig. 2 A/B) and clinically (Fig. 2 D). When animals received a subcutaneous booster injection of TT prior to the transplantation of TTsynMOs (2.5x10⁶), the collateral vessel growth was augmented tremendously in comparison to the control stimulated cells and the saline injection.

Fig. 2C A comparison of blood restoration 3 weeks after ligation of the femoral artery, when the most significant differences were seen between groups. When MOs were engineered only with TT, without prior booster injection of TT and thus without an initial immune response, the collateral vessel growth increased mildly in comparison to saline injection and control stimulated synMOs.

Fig. 3A Histological examination of capillary density within the gastrognemius muscle before and up to 3 wks after ligation in untreated control animals.

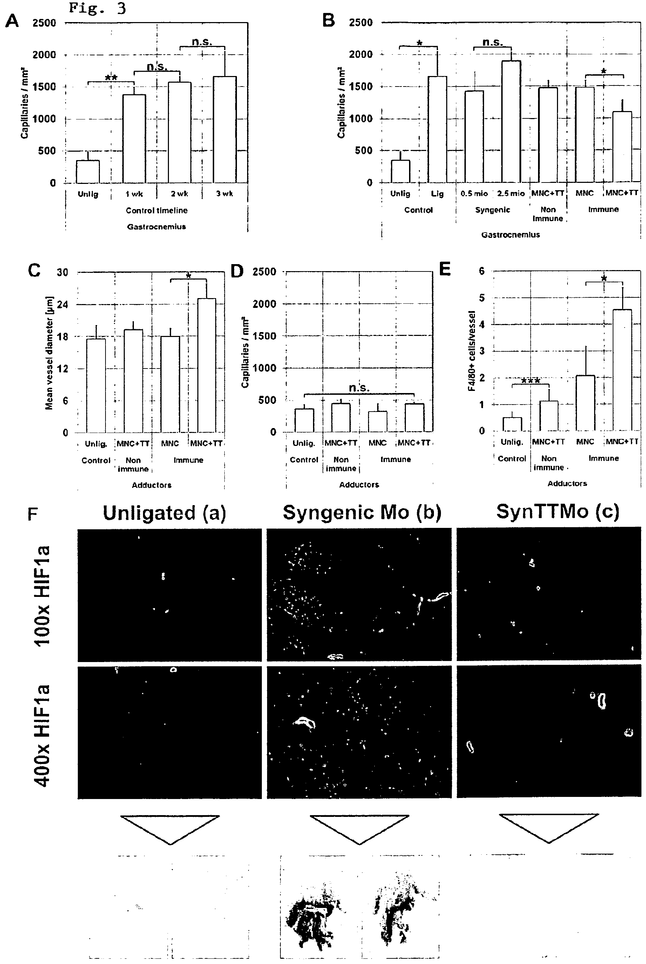

Fig. 3B Capillary density within the gastrognemius muscle 3 wks after ligation to compare the capillary sprouting between different experimental groups, demonstrate a significant decrease in capillary density after transplantation of synTTMOs indicating better blood supply to areas of ischemia (lower limb) by augmentation of nutritive collaterals within the upper limb. This is supported by an increase in mean vessel diameter of collateral arteries found within the adductor muscle (Fig. 3 C). Only transplantation of TTsynMOs increased vessel diameter significantly. Data represent mean ±s.d. of five mice per group. *P < 0.05.

Fig. 3D Changes in capillary spouting were not seen in the adductor muscle.

Fig. 3E Quantification of perivascular MO infiltration after systemic cell transplantation. Most infiltration of monocytes and macrophages around growing collaterals in the adductor muscle was seen when TTsynMOs had been transplanted into immunized mice.

Fig 3F Unligated leg (a) shows low levels of HIF1α and physiological perfusion within the distal foot.

Large bright spots: α-actin FITC staining, small bright spots: HIF1 α Cy3.

Immune histochemistry detection of HIF1α three weeks after ligation and transplantation of non-engineered synMOs revealed prolonged severe hypoxia in the calf of BALB/c mice, indicating increased capillary density as the major cause of ischemia driven angiogenesis (b). Poor clinical outcome after no transplantation or transplantation of non-engineered synMOs results in gangrene and necrosis (b; lower panel). After transplantation of TTsynMOs into immunized mice, HIF1α could not be detected in the thigh region and both capillary and vascular density remained unaltered, indicating normoxic conditions in the lower limb. This finding was confirmed by the clinical outcome (c).

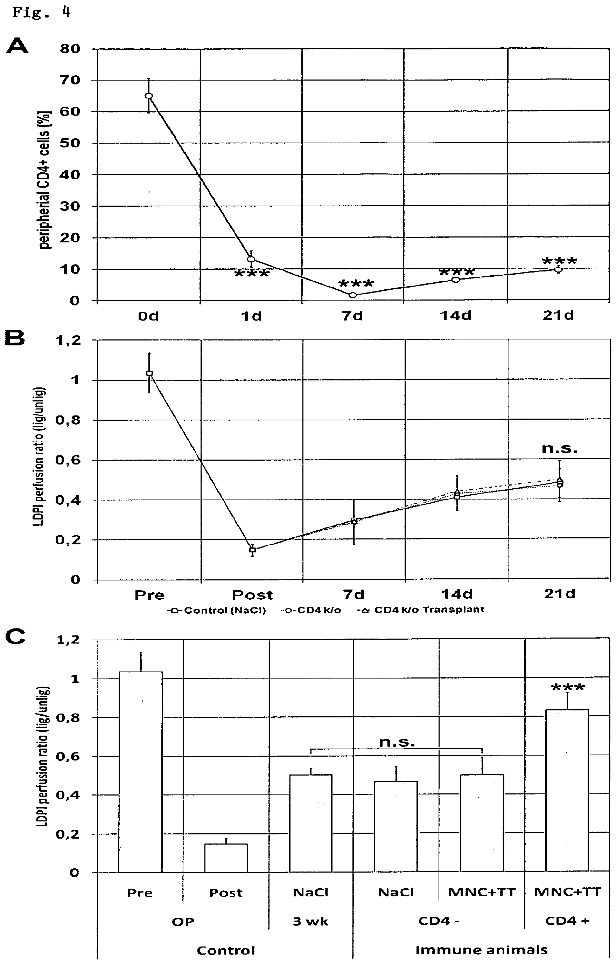

Fig 4A Graphical follow-up of alteration in blood cell fractions. Whereas concentration of MO remains unaltered, the CD 4 T cell fraction decreases dramatically after anti CD 4 antibody injection.

Fig. 4 B/C LDPI measurement of hind limb perfusion shows impairment of the positive effect of TTsynMO transplantation in promoting arteriogenesis after CD4 antibody application.

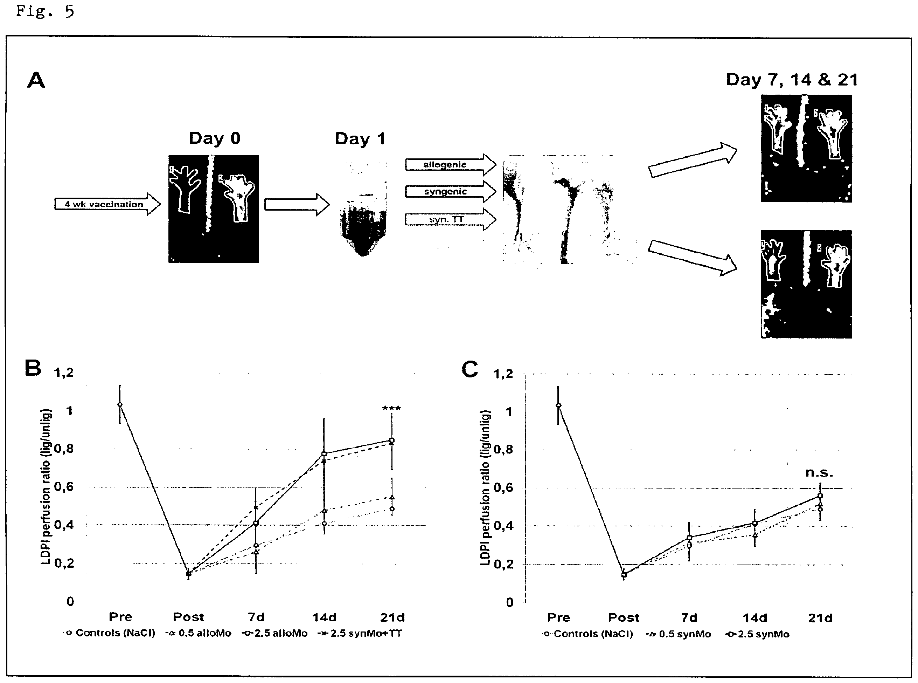

Fig. 5 A: Study design.

Fig. 5B Arteriogenic potential of allogeneic MO transplantation: Murine MOs were generated from bone marrow of BALB/c (for syngeneic transplantation) and C57BL/6 mice (for allogeneic transplantation).Transplantation of 0.5 x 10⁶ synMOs into BALB/c mice 24 hours after ligation of the hind limb only resulted in a mild increase of vascularisation (PI= 0.51 ±0.08 vs. saline injection PI= 0.48±0.09, n.s.).

Increasing the number of synMOs up to 2.5 x 10⁶ did not result in a further increase of revascularisation (PI= 0.56±0.06; n.s.). While transplantation of 0.5x10⁶ al-MOs resulted only in a mild increase in PI (PI=0.5±0.1; n.s.), transplantation of 2.5x10⁶ al-MOs demonstrated a 75% increase of collateralisation (PI 0.85±0.14 vs. control 0.48±0.09, P<0.001).

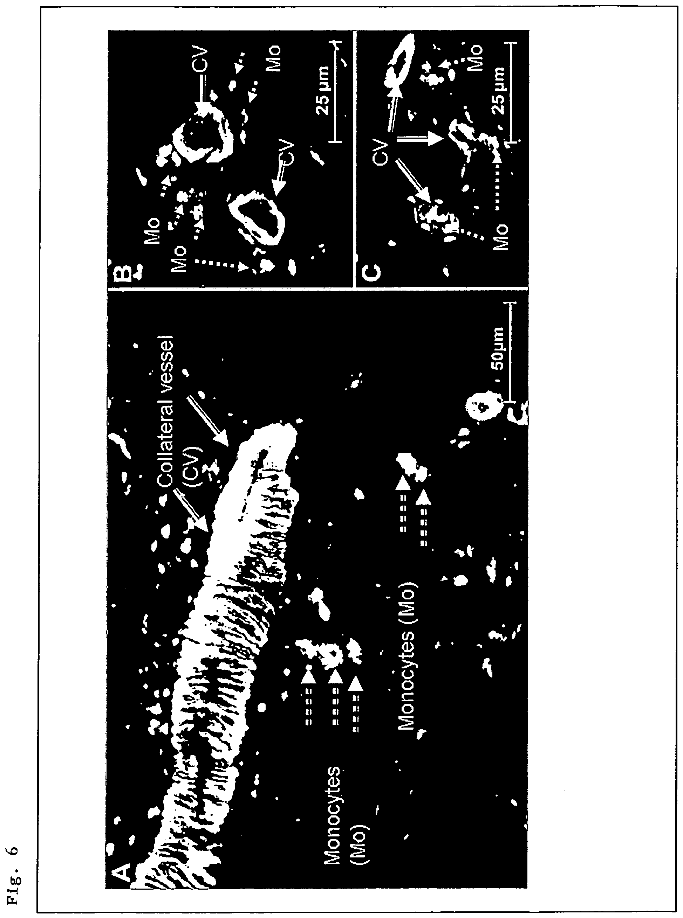

Fig. 6: Histological examination of perivascular MO infiltration after systemic cell transplantation. MO infiltration was assessed by counting F4/80 positive cells near vessels identified as arteries by their α-actin staining.

### Examples

### A) Materials and Methods

The present experiments were performed with permission of the State of Saxony, Regierungspraesidium Dresden, according to Section 8 of the German Law for animal protections. 8- to 13-week-old male BALB/c/and C57BL/6 mice from Charles River Laboratories (Sulzfeld, Germany) were examined. Mice were anaesthetised by intraperitoneal injection of xylazine (20 mg/kg body weight) and ketamine (110 mg/kg body weight, MEDISTAR GmbH, Holzwickede, Germany). The left femoral artery of BALB/c mice was occluded by ligation with 6-0 silk sutures immediately distal to the origin of the deep femoral artery and proximal to the origin of any other distal branch. Hind limb perfusion was determined by laser Doppler perfusion imaging (LDPI) (PeriMed, Stockholm, Sweden). The mean values from the occluded and non-occluded side were divided to calculate a perfusion index (PI). LDPI was carried out before, immediately after, 7, 14 and 21 days following occlusion (N=7). Histological quantification of vessel density was done on day 21 (N=3).

### Isolation and differentiation of murine bone marrow-derived monocytes (BMDMs)

A protocol published by Herold, J. et al., J Histochem Cytochem 59, 813-825 (2011).
was used: bone marrow cell suspensions were isolated by flushing femurs and tibias of 8-12 week-old BALB/c and C57BL/6 mice (Charles River, Sulzfeld, Germany) with complete RPMI 1640 (supplemented with 10% FCS, 1% Pen/Strep; PAA, Pasching, Austria). Aggregates were dislodged by gentle pipetting and debris was removed by passaging the suspension through a 70 µm cell strainer. Cells were washed twice with medium, adjusted to a suspension of 10⁶ cells/ml and seeded on 6-well ultra-low-attachment surface plates (Corning Costar, Schiphol-Rijk, Netherland). Cell cultures were supplemented with 20 ng/ml rmM-CSF (Miltenyi, Bergisch Gladbach, Germany) and cultured in a humidified incubator at 37 °C and 5% CO2 for five days. Cells were harvested at indicated time points by gentle pipetting and repeated washing of the wells with PBS, 0.5% BSA and 2 mM EDTA to detach adherent cells. Before transplantation, cell suspensions were depleted of CD117+ cells by MACS technology (Miltenyi, Bergisch Gladbach, Germany) and phenotypically and functionally characterised by FACS analysis (Supplementary Fig A).

### FACS analysis

Cell suspensions were harvested by gentle pipetting with EDTA-containing media (PBS+0.5% BSA+2mM EDTA), and stained with antibodies conjugated to Alexa or PE fluorochrome for 20 min at 4°C. The following antibodies were used: CD4, CD8a, CD11b, CD11c, CD25, CD45, CD45R, CD80, CD86, CD115, Gr-1, CD154, MHC class II receptor (all from eBioscience, San Diego, USA), F4/80 (AbD Serotec, Duesseldorf, Germany) or CD117 (c-kit) (Miltenyi Biotec, Bergisch Gladbach, Germany). MOs were collected by isolating CD115+, CD11b+, Gr-1+ and F4/80low cells as described previously

Herold, J.et al., J Histochem Cytochem 59, 813-825 (2011). Appropriate unspecific isotypes were used as negative controls (eBioscience, San Diego, USA). Samples were analysed on a FACS Calibur (Becton Dickenson, San Jose, USA) using CellQuest (Becton Dickenson, San Jose, USA). The phenotype of isolated MOs was further verified by detecting MHC class II receptor up-regulation and antigen presentation. Functional phenotyping of isolated MOs was done by static and dynamic adhesion to endothelial cells in a fluid chamber as described previously.

### Ex vivo cell engineering by tetanus toxin

MOs were co-stimulated with INF-y to increase MHC class II receptor upregulation and thereby improve CD4-T-cell activation via antigen presentation.

MOs were stimulated with either 8 ng/ml mrINF-γ (eBioscience, San Diego, USA) and 100 µg/ml or TT alone for 24 h and harvested. MOs were stimulated with either 8 ng/ml mrINF-γ alone or 100 µg/ml TT plus 8 ng/ml mrINF-γ (eBioscience, San Diego, USA) for 24 h and harvested. MHC class II receptor expression was quantified by FACS analysis. MOs were gated according to expression of F4/80.

### Cell transplantation

MOs were harvested, resuspended in saline and injected into the tail vein of mice for systemical application. The volume of either the cell suspension or saline without cells was 300 µl. Cell transplantation was done 24 h after ligation of the femoral artery. To control for possible contamination by remaining stem and progenitor cells, MOs for control and engineering experiments were taken from the same isolation pool.

CD4 T-cell depletion: To deplete CD4-cells, a specific anti-CD4 antibody (100µg, eBioscience, San Diego, USA) was injected intraperitoneally before and 24 h after ligation of the femoral artery. CD4-deplention was verified by longitudinal FACS analyses of mouse serum.

### Immunisation of mice

Immunization and booster injection was done with commercially available tetanus toxoid (Tetanol pur®, Novartis, Marburg, Germany). To immunize the mice 8 I.E. TT was used in a volume of 100 µl saline. TT was injected subcutaneously into the neck of the mice at day 0; a booster injection was given three weeks later by intraperitoneal injection of 100 µl TT. To determine the immunological response, 120 µl blood serum samples were taken before TT-immunization, two weeks afterwards and one week following the booster injection. Blood samples were stored at -20 °C for later analysis of anti-TT-antibody levels and production of TT titer by ELISA (Institute Virion/Serion, Freiburg, Germany). These measurements were validated by anti-mouse-IgG analysis (anti-mouse-IgG, Sigma Aldrich, Hamburg, Germany).

Leukocytes were isolated from immunized and non-immunized mice and prepared according to standard protocols. Briefly, cell suspensions were adjusted to give a density of 5 x 10⁶ cells/ml for cytokine secretion or 2.5 x 10⁶ cells/ml for proliferation assays in complete DMEM high glucose medium (Sigma Aldrich, Hamburg, Germany). To each 24-well of leukocytes, 7.5 x 10⁶ MOs were added in different conditions: MOs + 20 µg/ml TT, MOs + 100 µg/ml TT, MOs + 8 ng/ml INF-y, MOs + 20 µg/ml TT + 8 ng/ml INF-y, MOs + 100 µg/ml TT + 8 ng/ml INF-y, and un-stimulated MOs. The medium was supplemented with 20 ng/ml rmM-CSF (Miltenyi, Bergisch Gladbach, Germany) for a 24 h pre-stimulation period. Supernatant from the co-cultures was stored at -80 °C after a 48 h incubation period to quantifycytokine production by ELISA (eBioscience, San Diego, USA).

To determine CD4-Cell proliferation, 2 x 10⁶/ml spleen-derived leukocytes were incubated in co-culture with 7.5 x 10⁶ MOs for 5 days supplemented with carboxyfluorescein-diacetat-succinimidyl-ester (CFSE, Invitrogen, Karlsruhe, Germany). Cells were harvested after five days and counterstained with Alexa 647 anti-CD4 (Sigma Aldrich, Hamburg, Germany) before quantification of CD4 T-cell proliferation by FACS analysis. Proliferating CD4 cells were quantified based on the dilution of CFSE fluorescence and co-expression of CD4.. Endogenous CD4 T-cell proliferation capacity was analyzed and termed "baseline proliferation". To induce lymphocyte interaction above this baseline level either pure TT (20 µg/ml) or TT-engineered MOs were added to spleen derived T-cells from TT-pre-immunized mice.

Proliferation was also determined by a MTT (3-(4,5-Dimethylthiazol-2-yl)-2,5-diphenyltetrazolium bromide) assay. Cells were incubated with MTT for 3 h and cell number determined by ELISA (Cell growth-determination kit, Sigma Aldrich, Germany). Clonal cell expansion was abolished by co-incubation of the cultures with blocking functional grade anti-MHC class II receptor antibodies (clone M5/114.15.2, final concentration 2 µg/ml, MHC class II receptor I-A/I-E CD4L M5/114.15.2 Rat IgG2b, eBioscience, San Diego, USA).

### In vitro lymphocyte stimulation and proliferation assay Cell tracking of MO homing to areas of collateralisation

MOs were incubated with 5 µg/ml acLDL and stained with Dil fluorochrome (Invitrogen, Karlsruhe, Germany) for 24 hours. Histology was done 48 h after systemic MO transplantation for homing experiments. FITC anti-α-actin and KI 67 staining were used to detect proliferation of growing collaterals. A specific F4/80 antibody (eBioscience, San Diego, USA) was used to indentify MO infiltration in areas of collateralisation.

### Immune fluorescence and morphometrical analysis

Mice were sacrificed at indicated time points and hind limb muscles were embedded in optimal cutting temperature freezing medium (OCT) and frozen in liquid nitrogen cooled 2-methylbutane. Samples were stored at -80 °C. 5- to 7-µm cross sections were taken on a CM 1900 cryotome (Leica, Wetzlar, Germany) and air dried before being refrozen at -20 °C. Samples were further processed by rehydration and repeated washing in PBS, fixation and permeabilization in ice cold acetone. Blocking was done with serum free protein block (Dako, Hamburg, Germany) for 20 minutes. Sections were incubation with primary antibodies at recommended dilutions (rabbit anti-CD31 and rabbit anti-Ki67, Abcam, Cambridge, UK); rat anti-F4/80, eBioscience, San Diego, USA) for 1 h at room temperature, followed by incubation with an appropriate cross-absorbed secondary antibody conjugated to Cy3 fluorochrome. A FITC-conjugated mouse anti-mouse-SMC-α-actin antibody (Sigma Aldrich, Hamburg, Germany) and KI 67 staining were used to identify growing collaterals, followed by nuclear counterstaining with DAPI. Sections were analysed on an AxioverS100 (Carl Zeiss, Jena, Germany) and a RT/SE camera system (Diagnostic Instruments, Sterling Heights, USA). Capillary density was measured at 400x magnification of CD31-stained sections, vessel-size and -density were analyzed at 200x magnification of a α-actin-stained tissues; only vessels with a media-to-lumen-ratio typical for arteries were taken into consideration. MO infiltration was assessed by counting F4/80 positive cells near vessels identified as arteries by their α-actin staining.

### Cytokine quantification by Luminex technology

To determine the immune response and regulation of the inflammatory reaction that boosts collateral vessel growth dramatically, cytokine, chemokine, and growth factor levels were measured in mice serum at different time points (Blood sample time line: before op, +8h, +24h, +72h, +7d, +14d, BE +21d. Serum was stored after a spin down procedure at -80°C, mice had been injected 250 µl saline i.p.. Mouse serum was collected and cytokine concentrations of IL-1 alpha, IL-1beta, IL-2, IL-3, IL-4, IL-5, IL-6, IL-9, IL-10, IL-12 (p40), IL-12 (p70), IL-13, IL-17, Eotaxin, G-CSF, GMCSF, IFN-gamma, KC, MCP-1 (MCAF), MIP-1alpha, MIP-1beta, RANTES, TNF-alpha and FGF basic, IL-15, IL-18, LIF, MCSF, MIG, MIP-2, PDGF-BB, and VEGF were determined using the multiplex pro mouse cytokine 23-plex and 9-plex panel and the cytokine reagent kit (Bio-Rad Laboratories, Hercules, CA, USA) according to the manufacturer's protocol and as described previously. Samples were analyzed as triplets and standards in duplicates, using a Luminex-100 instrument with Bio-Plex Manager 4.1 software (Bio-Rad Laboratories).

### Statistical analyses

Statistical analysis was carried out using Microsoft Excel and IBM SPSS 16. Data was tested using standard T-test for parametrical data or Mann-Whitney U-test for non-parametrical data. When multiple testing was performed, p values were Bonferroni-adjusted. P-values were considered significant when p<0.05 (*), p<0.01 (**) or p<0.001 (***).

### B) Example 1:

### 1.1 TT antibody concentration increased after tetanus toxoid immunization of donor mice

To induce a cellular immune response in mice serving as source of MOs for the cell transplantation experiments described below, BALB/c mice were actively immunized with tetanus toxoid (TT). To assess this immune response, the serum tetanus titer was determined by ELISA. Concentrations increased from non detectable on baseline to 34±13 µg/ml after 14 days and 173±11 µg/ml after 28 days, respectively (Fig. 1). Antibody titers were measured by serum-titration and reached 1:1000±100 after 14 days and 1:9500±2100 after 28 days, respectively (Fig. 1 B). Iindicating an appropriate immune response against TT.

### 1.2 Syngeneic monocytes engineered ex vivo to present tetanus toxoid

First, syngeneic MOs (synMOs) from the bone marrow of either TT-immunized or non-immunized (control) BALB/c mice were generated by harvesting cells and culturing them in RPMI medium supplemented with M-CSF as described in the methods. To increase expression of MHC class II antigens by these synMOs and thereby enhance their ability to activate CD4 T cells, a co-stimulation with INF-y in vitro was done. The degree of stimulation was measured by extensive FACS phenotyping, analysis of MHC II upregulation and MHC II antibody experiments. CD11b, CD11c, CD45, CD62L, CD115, Gr-1 and F4/80 expression did not change in the co-stimulated synMOs, indicating that their differentiation and maturation were not influenced by INF-γ. However, the activation state appeared effected as CD80 and CD86 expression slightly increased.

Based on these findings, all antigen uptake and presentation assays were conducted under INF-γ co-stimulation.

Next, the synMOs were engineered to present TT antigen by exposing them to 100 µg TT for 24 h. Analysis of antigen presentation showed an increase of MHC class II receptor expression from 23±11% (endogenous MHC II expression) in non-stimulated MOs to 61±12% after 24 h exposure to INF-γ and to 88±12 after 24 h stimulation with INF-γ and TT. Thus, synMOs were successfully engineered ex vivo to present TT antigen prior to transplantation.

### 1.3 TT-engineered MOs induce T cell proliferation

To determine if leukocytes from TT-immunized mice recognize TT-engineered syngeneic MOs (TTsynMOs), T cell expansion and cytokine secretion was quantified following co-culture. Spleens were harvested from immunized and non-immunized BALB/c mice to isolate leukocytes. FACS analysis revealed that the resulting cell suspension contained 27.9±5.4% CD4 cells; 14.2±3.6% CD8 cells; 46±4.4% B-cells; and 3.2±0.8% CD4+ CD25+ cells (Fig.1 C) 8% of the cells were positive for CD11b+, which marks MOs and polymorphonuclear leukocytes (PMNs). Cell viability was > 90% as determined by propidium iodide. Fig 1 D: T-cell expansion was analyzed after stimulation with either TTsynMO or non-engineered synMOs. Using an MTT-assay, it was found the proliferation of T-cells isolated from TT-immunized mice increased by more than 200% after co-incubation with TTsynMOs compared with non-engineered synMOs (this endogenous proliferation was, termed baseline). Baseline-proliferation increased 1.3+/-0.1 fold after adding monocytes to the splenocyte culture (p<0.05). When monocytes where loaded with tetanus antigen, proliferation increased 2.6+/-0.2 fold compared to baseline (p<0.01 vs. baseline, p<0.05 vs. unloaded monocytes) (Fig. 1 D). Proliferation was attenuated to values similar to baseline non-stimulated MOs and tetanol pur injection when a specific antibody against MHC class II receptor was added to the cell suspension, indicating that the proliferation was a specific response to antigen presentation by the TTsynMOs. Together these in vitro data show that TTsynMOs induce T cell proliferation in leukocytes from TT-immunized mice.

### Example 2:

### TT-engineered syngeneic MOs communicate with CD4 T cells

To detect whether TTsynMOs communicate with CD4 T cells, the expansion of all leukocytes using a CFSE proliferation assay was analyzed. CD4 T cells were specifically visualized by an anti-CD4-antibody. It was found that CD4 cell proliferation increased up to 1.5 fold following incubation with TTsynMOs compared with endogenous T cell proliferation (baseline) and non-stimulated MNC (Fig. 1 E). This suggests that, as expected, TTsynMOs act via a CD4 T cell dependent pathway to elicit a host response.

### Example 3:

### Immunization with TT induces B cell memory and IL-2 secretion

To determine whether immunization of host mice induces a T cell-dependent immune response TTsynMOs were cultured with host T cells. As a predictor for B cell activation, and thus antibody production, the level of IL-2 in the supernatant of spleen-derived T cells from pre-immunized mice was analyzed with a commercially available sandwich ELISA. When spleen-derived T cells from immunized mice were co-cultivated with TTsynMOs, IL-2 production increased dramatically compared with endogenous T cell IL-2 production (baseline level, 208±207 pg/ml to 1557±425 pg/ml, p<0.001 Fig. 1F). This was superior to stimulation of the TT-immunized T cells by adding TT directly to the cell culture (816±362 pg/ml vs. baseline 208±207 pg/ml, p<0.01). Remarkably, non-engineered synMOs also induced a strong increase (897±357 pg/ml, p<0.001), suggesting unspecific lymphocyte activation. These data demonstrate massive B cell proliferation and increased IL-2 production, indicating that the antigen presenting capacity of TTsynMOs to T and B cells is intact. Additionally, it was shown that this occurs through a CD4 T cell-dependent pathway because addition of MHC II antibodies abolished the effect of IL-2 secretion.

### Example 4:

### TT-engineered syngeneic MOs augment blood flow in the ligated hind limb

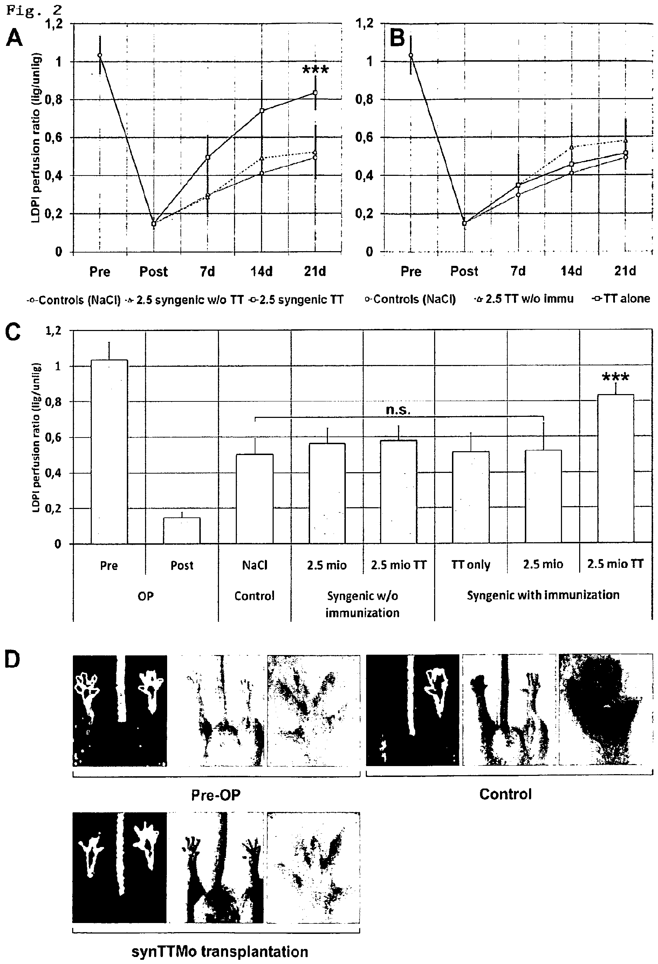

To test the effect of MO transplantation in a clinically relevant model, either saline, synMOs, or TTsynMOs were injected intravenously into the tail vein of BALB/c mice 24 h after ligation of the femoral artery. Hind limb perfusion was quantified by laser Doppler perfusion imaging. The perfusion index (PI) in saline injected mice revealed that restoration of the blood supply after ligation was insufficient (Fig 2A-o- 0.16±0.04 directly post-ligation; 0.29±0.08 after 1 wk; 0.41±0.07 after 2 wks and 0.48±0.09 after 3 wks, all p<0.001). 80% of the mice suffered from hind limb necrosis and gangrene (Fig. 2 D). The most striking difference in perfusion between the groups was seen 3 wks post- ligation (Fig. 2C). Transplantation of 2.5x10⁶ synMOs only mildly increased vascularization (PI=0.57±0.08 vs. saline PI=0.48±0.09 n.s.. These data provide evidence that it is possible to augment collateral vessel growth by triggering an adaptive immune response in which activated CD4 T cells induce an inflammatory response that in turn induces collateral vessel growth.

It was tested whether augmentation of collateral vessel growth depends on activation of an adaptive immune response in which memory T cells and antibody production by B cells recall an immune response against the TTsynMOs. For this set of experiments, the host mice were immunized with TT prior to receiving the transplant. No adverse effects of active immunization, such as fever, malaise or discomfort were seen. It was found that transplanting 2.5x10⁶ TTsynMOs into pre immunized mice tremendously increased collateralization compared with transplantation of either synMOs or TTsynMOs into non-immunized mice (PI: TTsynMOs (pre-imm) 0.83±0.09 vs. TTsynMOs (non immunized) 0.59±0.14 vs. saline 0.48±0.09, P<0.001;. Transplantation of TTsynMOs into immunized mice restored hind limb perfusion to high levels, similar to the transplantation of allogenic MOs (PI: TTsynMOs 0.83±0.09 vs. alMOs 0.85±0.14 vs. saline 0.48±0.09, P<0.001; Fig. 5 B). Implantation of 2.5 x 10⁶ cells had the maximal effect with no further increase seen using greater cell numbers (Fig. 5 B). Animals did not develop ischemic syndromes when 2.5 x 10⁶ TTsynMOs were injected. Thus immunization of the host dramatically increases the arteriogenic effect of TTsynMOs transplantation.

The possibility that the pro-arteriogenic effect of the cell therapy is mediated solely by unspecific MO activity, rather than dependent on T cell memory was ruled out. Although the present TTsynMOs actively present TT antigen, injecting these cells into a non-immunized host, which had no initial immune response, only mildly increased collateral vessel growth compared with the control (PI 0.59±0.09 vs. control stimulated (only INF-γ) 0.56±0.06 vs. saline 0.52±0.13, P<0.001) (Fig. 2 C). This is consistent with TTsynMOs interacting with host CD4 T cells that then mediate the immune response driving collateral vessel growth. It is unlikely that such a response could be generated solely by a diffuse acute inflammatory immune reaction to foreign MOs. Additionally it was found that presenting TT antigen directly to the immune system of immunized mice by subcutaneous injection had no arteriogenic effect PI=0.51±0.11 vs. saline 0.52±0.13, P<0.001, n.s. Fig B -x-). This indicates that the antigen presentation capacity of the synMOs is tremendously important for this cellular therapy.

### Example 5:

### TT-engineered syngeneic MO transplantation reduce ischemia within the lower limb

An endogenous increase in capillary density within the calf muscle of non treated mice from 350±130 to 1450±110 capillaries/mm² after one week (p< 0.01), 1570±90 after two weeks (p< 0.05 vs. one week), and 1660±390 capillaries/mm² after three weeks (p < 0.001) (Fig. 3 A) was found. The response at week 3 between the different treatment groups were compared because it was the greatest (Fig. 3B). Capillary density and MO infiltration were analyzed histologically following transplantation of TTsynMOs into immunized hosts. Distal capillary formation was significantly reduced when recipients had received TTsynMOs (2.5x10⁶), implying that arteriogenesis resulted in sufficient blood supply to the distal limb (1100±180 vs. 1660±390 untreated mice vs. 1900±200 synMOs capillaries/mm², p<0.05 (Fig. 3B). This finding was supported by a 12% increase in arteriolar diameter in this group in areas of collateralization (upper limb) (Fig. 3 C). Capillary density in the adductor muscle was not altered in any group (Fig. 3 D).

Immunohistochemical visualization of HIF1α three weeks after ligation was used as an additional marker of ischemia within the distal gastrocnemius muscle. Three weeks after ligation of the hind limb in BALB/c mice, HIF1α was still visible, consistent with persistent local ischemia triggering angiogenesis (Fig. 3F). However, when TTsynMOs were transplanted, HIF1α staining was almost abolished suggesting normoxic conditions in the lower limb (Fig.3F, right panel). Perivascular inflammation within the proximal adductor muscle was evaluated, where collateralization is best visualised. Compared to the unligated leg, a pronounced perivascular monocyte accumulation in the ligated leg of non-immune animals that were transplanted with TTsynMOs (0.5±0.5 vs. 1.1±0.7 cells/vessel) was found. In immune animals, the transplantation of TTsynMOs lead to an even more accentuated monocyte accumulation also than in (4.5±1 vs. 2.0±1.1 vs., p<0.05) (Fig. 3 E).

### Example 6:

### Arteriogenic response to MO transplantation is CD4 T cell dependent

It was tested whether transplantation of TTsynMOs augments collateralization through a CD4-dependent T cell response. BALB/c mice were depleted of CD4 cells by an antibody treatment that reduced native CD4 T cells by 90.2% after 2 wks (Fig.4 A). Blood samples were taken before and after ligation and transplantation of TTsynMOs. Although the concentration of MOs in the blood was almost unchanged over 21 days (also after theoretically increasing their number by transplantation, Fig. 4 A), a rebound of granulocyte numbers following ligation and TTsynMOs transplantation was observed. This supports that injected TTsynMOs home to areas of collateralization then induce local inflammation recall by host granulocytes. When TTsynMOs were injected after CD4 T cell depletion, the arteriogenic response was completely abolished and similar to perfusion in control animals (Fig. 4 B: PI after transplantation of engineered MOs 0.49±0.9 vs. PI after depletion + ligation without transplantation; 0.46±0.08 vs. only ligation + no CD4 depletion 0.5±0.03, (p<0.001) Fig. 4 C). These data suggest that the immunological response of the host to the graft is CD4-dependent.

### Example 7:

### Homing of transplanted monocytes

Homing of the transplanted TTsynMOs by labelling them ex vivo with Dil-LDL was observed, then visualizing them immunohistochemically in areas of collateralization (Fig. 6). TTsynMOs contribute directly to the remodelling of collateral arteries.

## Claims

1. An antigen-loaded antigen-presenting cell (APC) for use in treating a disease selected from the group consisting of coronary artery diseases, cerebral-occlusive diseases, peripheral-occlusive diseases, visceral-occlusive diseases, renal artery diseases and mesenterial arterial insufficiency in a subject in need thereof, wherein the subject in need thereof is a subject which has been immunised against said antigen.

2. The antigen-loaded APC for use according to claim 1, wherein the subject is a human subject.

3. The antigen-loaded APC for use according to any one of the preceding claims, wherein the APC is a monocyte, a monocyte precursor or a dendritic cell.

4. The antigen-loaded APC for use according to any one of the preceding claims, wherein the antigen is tetanus toxoid, pertussis toxin or diphtheria toxin.

5. The antigen-loaded APC for use according to any one of the preceding claims, wherein said APCs are autologous or syngeneic to the subject.

6. The antigen-loaded APC for use according to any one of the preceding claims, wherein the collateral vessel are collateral arteries or arteries from pre-existing arteriolar connections.

7. The antigen-loaded APC for use according to any one of the preceding claims, wherein the subject in need thereof is subjected or was subjected to an agent or radiation or surgical treatment damaging or destroying arteries.

8. A pharmaceutical composition for use in treating a disease selected from the group consisting of coronary artery diseases, cerebral-occlusive diseases, peripheral-occlusive diseases, visceral-occlusive diseases, renal artery diseases and mesenterial arterial insufficiency in a subject in need thereof comprising an antigen-loaded APC, wherein the subject in need thereof is a subject which has been immunised against said antigen and a pharmaceutical acceptable carrier.

## Patentansprüche

1. Antigenbeladene, antigenpräsentierende Zelle (APZ) zur Verwendung in der Behandlung einer Erkrankung ausgewählt aus der Gruppe bestehend aus koronaren Gefäßerkrankungen, zerebralen Verschlusserkrankungen, peripheren Verschlusserkrankungen, viszeralen Verschlusserkrankungen, Nierenarterienerkrankungen und mesenterialer arterieller Insuffizienz in einem Subjekt, das einen entsprechenden Bedarf aufweist, wobei das Subjekt, das einen entsprechenden Bedarf aufweist, ein Subjekt ist, das gegen das Antigen immunisiert wurde.

2. Antigenbeladene APZ zur Verwendung nach Anspruch 1, wobei das Subjekt ein menschliches Subjekt ist.

3. Antigenbeladene APZ zur Verwendung nach einem der vorhergehenden Ansprüche, wobei die APZ ein Monozyt, ein Monozytenvorläufer oder eine dendritische Zelle ist.

4. Antigenbeladene APZ zur Verwendung nach einem der vorhergehenden Ansprüche, wobei das Antigen Tetanus-Toxoid, Keuchhusten-Toxin oder Diphtherie-Toxin ist.

5. Antigenbeladene APZ zur Verwendung nach einem der vorhergehenden Ansprüche, wobei die APZs zu dem Subjekt autolog oder syngenetisch sind.

6. Antigenbeladene APZ zur Verwendung nach einem der vorhergehenden Ansprüche, wobei die Kollateralgefäße Kollateralarterien oder Arterien aus bestehenden arteriolären Verbindungen sind.

7. Antigenbeladene APZ zur Verwendung nach einem der vorhergehenden Ansprüche, wobei das Subjekt, das einen entsprechenden Bedarf aufweist, einem Mittel, einer Bestrahlung oder einer chirurgischen Behandlung ausgesetzt ist oder war, das/die Arterien beschädigt oder zerstört.

8. Pharmazeutische Zusammensetzung zur Verwendung in der Behandlung einer Erkrankung ausgewählt aus der Gruppe bestehend aus koronaren Gefäßerkrankungen, zerebralen Verschlusserkrankungen, peripheren Verschlusserkrankungen, viszeralen Verschlusserkrankungen, Nierenarterienerkrankungen und mesenterialer arterieller Insuffizienz in einem Subjekt, das einen entsprechenden Bedarf aufweist, wobei die Zusammensetzung eine antigenbeladene APZ sowie einen pharmazeutisch akzeptablen Träger umfasst, wobei das Subjekt, das einen entsprechenden Bedarf aufweist, ein Subjekt ist, das gegen das Antigen immunisiert ist.

## Revendications

1. Cellule présentatrice d'antigène (CPA) chargée en antigène pour l'utilisation dans le traitement d'une maladie choisie dans le groupe constitué par les maladies coronariennes, les maladies occlusives cérébrales, les maladies occlusives périphériques, les maladies occlusives viscérales, les maladies des artères rénales et l'insuffisance artérielle mésentérique chez un sujet qui en a besoin, dans laquelle le sujet qui en a besoin est un sujet qui a été immunisé contre ledit antigène.

2. CPA chargée en antigène pour l'utilisation selon la revendication 1, dans laquelle le sujet est un sujet humain.

3. CPA chargée en antigène pour l'utilisation selon l'une quelconque des revendications précédentes, dans laquelle la CPA est un monocyte, un précurseur d'un monocyte ou une cellule dendritique.

4. CPA chargée en antigène pour l'utilisation selon l'une quelconque des revendications précédentes, dans laquelle l'antigène est le toxoïde de tétanos, la toxine pertussique ou la toxine diphtérique.

5. CPA chargée en antigène pour l'utilisation selon l'une quelconque des revendications précédentes, dans laquelle les CPA sont autologues ou syngéniques par rapport au sujet.

6. CPA chargée en antigène pour l'utilisation selon l'une quelconque des revendications précédentes, dans laquelle les vaisseaux collatéraux sont des artères collatérales ou des artères provenant de connexions artériolaires préexistantes.

7. CPA chargée en antigène pour l'utilisation selon l'une quelconque des revendications précédentes, dans laquelle le sujet qui en a besoin est ou a été soumis ou exposé à un agent, une irradiation ou un traitement chirurgical endommageant ou détruisant des artères.

8. Composition pharmaceutique pour l'utilisation dans le traitement d'une maladie choisie dans le groupe constitué par les maladies coronariennes, les maladies occlusives cérébrales, les maladies occlusives périphériques, les maladies occlusives viscérales, les maladies des artères rénales et l'insuffisance artérielle mésentérique chez un sujet qui en a besoin, la composition comprenant une CPA chargée en antigène et un support pharmaceutiquement acceptable, dans laquelle le sujet qui en a besoin est un sujet qui a été immunisé contre ledit antigène.
